(19) Europäisches Patentamt
European Patent Office
Office européen des brevets

(11) **EP 4 218 569 A1**

(12) **EUROPEAN PATENT APPLICATION**
published in accordance with Art. 153(4) EPC

(43) Date of publication:
**02.08.2023 Bulletin 2023/31**

(51) International Patent Classification (IPC):
**A61B 5/1455** (2006.01)

(21) Application number: **21871417.8**

(22) Date of filing: **17.09.2021**

(86) International application number:
**PCT/CN2021/118986**

(87) International publication number:
**WO 2022/063047 (31.03.2022 Gazette 2022/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.09.2020 CN 202011002263**

(71) Applicant: **Global Health Ark Medical Technology (Beijing) Co. Ltd.**
**Beijing 100083 (CN)**

(72) Inventors:
• **TANG, Fei**
**Beijing 100600 (CN)**
• **WU, Chenyang**
**Beijing 100600 (CN)**
• **GENG, Zhanxiao**
**Beijing 100600 (CN)**
• **QIU, Zan**
**Beijing 100600 (CN)**
• **WANG, Xiaohao**
**Beijing 100600 (CN)**

(74) Representative: **Worthington, Richard Easton**
**Withers & Rogers LLP**
**2 London Bridge**
**London SE1 9RA (GB)**

(54) **PHOTOPLETHYSMOGRAPHY-BASED NON-INVASIVE DIABETES PREDICTION SYSTEM AND METHOD**

(57) Disclosed is a photoplethysmography-based non-invasive diabetes prediction system, comprising an optical signal transmitter (10), a driving circuit (30), a receiving circuit (40) and a processor module (50). The optical signal transmitter (10), when driven by the driving circuit (30), obtains and emits an optical signal of a fixed wavelength and intensity. The receiving circuit (40) converts a received optical signal of photoplethysmography into an electrical signal of photoplethysmography, per-forms amplification, filtering, digital-analog conversion, and sends the digital photoplethysmography signal to the processor module (50). The processor module (50) performs signal processing, feature extraction, modeling and prediction on the digital photoplethysmographic signal, and visually displays same. The photoplethysmography-based non-invasive diabetes prediction system and method can achieve non-invasive, real-time and convenient diabetes screening and disease risk prediction.

FIG. 1

Processed by Luminess, 75001 PARIS (FR)

## Description

### Technical Field

The present invention relates to the technical field of medical health, in particular to a system and a method for non-invasive diabetes screening and disease risk prediction by means of optical sensors.

### Background Art

Diabetes is one of the most serious chronic metabolic diseases in the world. As of 2019, there are 460 million diabetic patients worldwide. It is predicted that the quantity of diabetic patients will increase to 700 million by 2045. Diabetes and its complications pose a heavy burden on individuals and the society. According to statistics, at present, about 50% of diabetic patients (mostly Type II diabetes) have not been diagnosed. Therefore, it is urgent to diagnose this undiagnosed population in order to provide appropriate care and prevent the disease from getting worse.

At present, glucose tolerance test is the "gold standard" for diabetes diagnosis, but this method requires the subjects to go to the hospital for invasive blood glucose collection, which is cumbersome and costly, limiting the accurate diagnosis of conditions of more diabetic patients.

The non-invasive diabetes screening methods employed at present focus on predicting the probability of diabetes by using the life data, case data or medical data of the subjects as the input. For example, the China Patent No. CN107680676A tilted as "Method for Predicting Gestational Diabetes Mellitus Based on Electronic Case History Data" and the China Patent No.CN106682412A titled as "Method for Predicting Diabetes Based on Medical Physical Examination Data". However, the methods have some shortcomings, such as difficulties in obtaining information and inconvenience in home use, and can't meet the requirements of daily diabetes health management.

### Summary of the Invention

In order to overcome the drawbacks in the prior art, the object of the present invention is to provide a photoplethysmography-based non-invasive diabetes prediction system and a method thereof, which can realize non-invasive, real-time and convenient diabetes screening and disease risk prediction by utilizing wearable red light-near infrared light sensor devices to acquire a pulse wave signal of human body.

To attain the above object, the photoplethysmography-based non-invasive diabetes prediction system provided by the present invention comprises an optical signal emitter, a driving circuit, a receiving circuit, and a processor module, wherein,

the optical signal emitter is driven by the driving circuit to obtain and emit an optical signal having a fixing wavelength and intensity;

the receiving circuit converts the optical signal of the received photoplethysmography signal into an electrical signal of the photoplethysmography signal, performs amplification, filtering, digital-analog conversion, and sends a digital photoplethysmography signal to the processor module; and

the processor module performs signal processing, feature extraction, modeling and prediction on the digital photoplethysmography signal, and visually displays the same.

Furthermore, the optical signal emitter further comprises a visible light transmitter having two or more wavelengths and a near-infrared transmitter having two or more wavelengths.

Furthermore, the receiving circuit comprises an optical signal receiver, an amplifier filter circuit, and an analog-digital converter circuit, wherein

the optical signal receiver converts the optical signal of the received photoplethysmography into the electrical signal of the photoplethysmography and sends the electrical signal of the photoplethysmography to the amplifier filter circuit;

the amplifier filter circuit performs amplification and filtering on the electrical signal of the photoplethysmography to eliminate high-frequency noises; and

the analog-digital converter circuit converts the electrical signal of the photoplethysmography into a digital photoplethysmography signal and then sends the digital photoplethysmography signal to the processor module.

Furthermore, the processor module eliminates low-frequency respiratory disturbances, obtains multifractal spectrum features from the digital photoplethysmography signal through wavelet transform, performs dimensionality reduction and clustering of feature space, establishes and trains a screening model, and performs prediction of new samples according to the screening model.

Furthermore, the processor module calculates multifractal spectrum coordinates and cumulative coefficients by using a wavelet transform modulus maxima (WTMM) method, and obtains multifractal spectrum features from the digital photoplethysmography signal.

Furthermore, the processor module extracts features from a generated photoplethysmography information database and performs normalization on the features, and performs principal component analysis on the feature space to realize feature dimensionality reduction; clusters the feature space after dimensionality reduction by K-means or KNN unsupervised learning , sets a number of clusters, logs the distance of each data item to the center of each cluster, and classifies the data according to the number of clusters.

Still furthermore, the processor module trains the screening model by using unsupervised learning and supervised learning in combination.

To attain the above object, the present invention further provides a photoplethysmography-based disease screening and risk prediction method, which comprises the following steps:

1) obtaining a pulse wave signal of the human body;

2) eliminating low-frequency respiratory disturbances from the pulse wave signal;

3) obtaining multi fractal spectrum features of the pulse wave signal on the basis of wavelet transform;

4) performing dimensionality reduction and clustering of feature space of the pulse wave signal;

5) establishing a screening model; and

6) performing prediction of new samples according to the screening model.

Furthermore, the step 3) further comprises calculating multifractal spectrum coordinates and cumulative coefficients by using a wavelet transform modulus maxima (WTMM) method.

Furthermore, the step 3) further comprises the following steps:

defining a wavelet transform function, and calculating a singular index of wavelet transform according to the pulse wave signal, a mother wavelet, and a scale factor;

setting a scale according to the scale factor, and calculating fractal dimensions having the same singular value according to the scale and the number of wavelet transform modulus maxima lines;

defining a segmentation function for wavelet transform modulus maxima, substituting the scale modulus maxima line set, the order and scale of statistical moments into the segmentation function, and calculating a scale function;

performing Legendre transform on the scale function, calculating the distribution of the segmentation function for wavelet transform modulus maxima at different scales, and performing least squares fitting to obtain the distribution of the moment corresponding to the scale function; and

performing Taylor expansion on the scale function to obtain a multifractal spectrum.

Furthermore, the step of performing Taylor expansion on the scale function to obtain a multifractal spectrum further comprises obtaining spectrum coordinates of fixed-step moment order, and using the spectrum coordinates and the cumulative coefficients as fractal features.

Furthermore, the step 4) further comprises generating a photoplethysmography information database, extracting features and performing normalization on the features, and performing principal component analysis on the feature space to realize feature dimensionality reduction.

Furthermore, clustering the feature space after dimensionality reduction by K-means or KNN unsupervised learning, setting the number of clusters, logging the distance of each data item to the center of each cluster, and classifying the data according to the number of clusters.

Furthermore, the step 5) further comprises establishing binary classification prediction models for the clustering results respectively.

Still furthermore, the step 6) further comprises weighting the distances of the features of the new samples to the centers of the clusters, and predicting a probability according to the weighting result.

The photoplethysmography-based non-invasive diabetes prediction system and method according to the present invention have the following beneficial effects:

1) The system and method can realize non-invasive diabetes screening, and are simpler and easier to use than the conventional invasive screening, and can be used for daily health management.

2) The system and method simply use several optical sensors to realize diabetes prediction based on multifractal features of a pulse wave signal, and employ compact hardware, thereby realizing a highly integrated and wearable design.

3) The system and method have a real-time detection advantage, and can present a diabetes prediction result within 1 minute.

Other features and advantages of the present invention will be detailed in the following description, and will become apparent partially from the description or be understood through implementation of the present invention.

**Brief Description of the Drawings**

The accompanying drawings are provided for further understanding of the present invention, and constitute a part of the specification. These drawings are used in conjunction with the embodiments of the present invention to interpret the present invention, but don't constitute any limitation to the present invention. In the figures:

FIG. 1 is a functional block diagram of the photoplethysmography-based non-invasive diabetes prediction system according to the present invention;

FIG. 2 is a flow chart of the photoplethysmography-based non-invasive diabetes prediction method according to the present invention;

FIG. 3 is a flow chart of implementing the photoplethysmography-based non-invasive diabetes screening and disease risk prediction method according to an embodiment of the present invention;

FIG. 4 is a schematic diagram of extracting respiratory disturbances from the pulse wave signal according to the present invention;

FIG. 5 is a schematic diagram of the pulse wave signal after the respiratory disturbances are removed according to the present invention;

FIG. 6 shows examples of multifractal spectrums of healthy persons and diabetic patients according to the present invention;

FIG. 7 is a schematic diagram of the clustering result when the number of clusters is 3 according to the present invention;

FIG. 8 is a ROC diagram of the accuracy of diabetes prediction according to the present invention.

**Embodiments**

Hereunder some preferred examples of the present invention will be described with reference to the accompanying drawings. It should be understood that the preferred examples described herein are only intended to describe and explain the present invention, but don't constitute any limitation to the present invention.

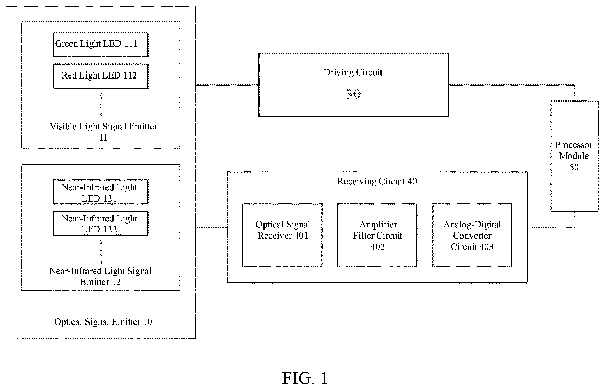

FIG. 1 is a functional block diagram of the photoplethysmography-based non-invasive diabetes prediction system according to the present invention. As shown in FIG. 1, the photoplethysmography-based non-invasive diabetes prediction system according to the present invention comprises an optical signal emitter 10, a driving circuit 30, a receiving circuit 40, and a processor module 50, wherein,

The optical signal emitter 10 is connected to the driving circuit 30 and the receiving circuit 40 respectively and configured to obtain a pulse wave signal of human body.

The processor module 50 is connected to the driving circuit 30 and the receiving circuit 40 respectively.

The processor module 50 controls the driving circuit 30 to act on the optical signal emitter 10, and the receiving circuit 40 receives the reflected or transmitted light from the optical signal emitter 10, the reflected or transmitted light is processed by photoelectric conversion, filtering and analog-digital conversion, and then is sent to the processor module 50.

In an embodiment of the present invention, the optical signal emitter 10 comprises a visible light signal emitter 11 and a near-infrared light signal emitter 12.

The visible light signal emitter 11 comprises two or more visible light LEDs, including a green light LED 111 with 530 nm wavelength and a red light LED 112 with 660 nm wavelength.

The near-infrared light signal emitter 12 comprises two or more near-infrared light LEDs, including a near-infrared light LED 121 with 805 nm wavelength and a near-infrared light LED 122 with 940 nm wavelength.

The visible light signal emitter 11 and the near-infrared light signal emitter 12 may be of a reflective type or transmissive type.

The driving circuit 30 is configured to drive the optical signal emitter 10 to transmit an optical signal.

The receiving circuit 40 processes photoelectric conversion and analog-digital conversion on the received optical signal, and then sends the processed signal to the processor module 50.

The processor module 50 is configured to perform signal processing, feature extraction, modeling and prediction on the multi-channel pulse wave signals, and visually display the same.

In an embodiment of the present invention, the processor module 50 eliminates low-frequency respiratory disturbances, and obtains multifractal spectrum features of the photoplethysmography signal through analysis of the wavelet; performs principal component analysis on the feature space to realize feature dimensionality reduction; trains a screening model by utilizing unsupervised learning and supervised learning in combination, and make prediction on new samples.

Preferably, the receiving circuit 40 comprises an optical signal receiver 401, an amplifier filter circuit 402, and an analog-digital converter circuit 403.

Specifically, the optical signal receiver 401 is connected to the amplifier filter circuit 402, and the amplifier filter circuit 402 is connected to the analog-digital converter circuit 403.

The optical signal receiver 401 is configured to receive an optical signal transmitted from the optical signal emitter and performs photoelectric conversion on the optical signal to obtain a pulse wave signal of human body.

The amplifier filter circuit 402 is configured to perform filtering on the received pulse wave signal of human body to eliminate high-frequency noises from the multi-band pulse wave signal of human body through low-pass filtering.

The analog-digital converter circuit 403 is configured to perform analog-digital conversion on the pulse wave signal of human body and sends a digital pulse wave signal of human body to the processor module 50.

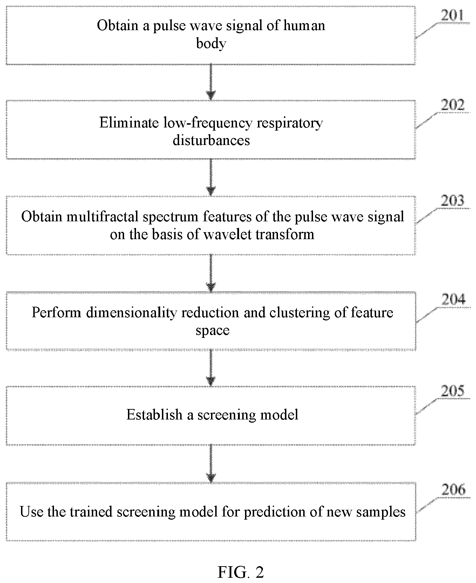

FIG. 2 is a flow chart of the photoplethysmography-based non-invasive diabetes prediction method according to the present invention. The photoplethysmography-based non-invasive diabetes prediction method provided by the present invention will be detailed below with reference to FIG. 2.

First, in step 201, a pulse wave signal of human body is obtained. In this step, a visible light-near infrared light sensor system is placed on the skin of a human body to obtain a pulse wave signal of human body. The sensor system comprises two or more visible light sensors and two or more near infrared light sensors.

In step 202, low-frequency respiratory disturbances are eliminated. In this step, low-frequency respiratory disturbances are obtained in the processor module 50 through smooth filtering according to the multi-band pulse wave signal, and the disturbances are removed from the raw signal to ensure that the DC component of the processed waves is smooth and steady.

In step 203, multifractal spectrum features of the pulse wave signal are obtained on the basis of on wavelet transform. In this step, multifractal analysis can describe the inherent law of a timing sequence waveform from a nonlinear aspect, and wavelet analysis is capable of characterizing the signal in the time domain and the frequency domain. Therefore, wavelet analysis is utilized to obtain multifractal features.

Preferably, multifractal spectrum coordinates and cumulative coefficients are calculated by using a wavelet transform modulus maxima (WTMM) method.

Preferably, the wavelet transform is set as follows:

$$W_\psi(t_0, a) = \frac{1}{a} \int_{-\infty}^{+\infty} f(t)\, \psi(\frac{t - t_0}{a}) dt \tag{1}$$

where $f(t)$ is the raw signal, $\psi(t)$ is a mother wavelet, and $a$ is a scale factor; it is proved that $W_\psi(t_0, a)$ is approximately

equal to $a^{h(t0)}$ when $a$ approaches to $0^+$, where $h(t_0)$ is a singular index and represents the singularity of the wavelet transform.

The scale function and the multifractal spectrum are estimated with a wavelet transform modulus maxima (WTMM) method. Denote $N_h(a)$ is the number of wavelet transform modulus maxima lines with scale = $a$ and singular value = $h$, then $N_h(a)$ can be expressed by:

$$N_h(a) = a^{-D(h)} \tag{2}$$

$D(h)$ is the fractal dimensions having the same singular value $h$.

Preferably, a segmentation function defining wavelet transform modulus maxima is defined as follows:

$$Z(q,a) = \sum_{l \in L(a)} |W_\psi(t_0,a)|^q \sim a^{\tau(q)} \tag{3}$$

$L(a)$ represents a set of modulus maxima lines with scale = $a$, $\tau(q)$ is the scale function, $q$ is the order of statistical moment; then, the following equation is obtained by substitute $N_h(a) = a^{-D(h)}$ into the above equation:

$$Z(q,a) \sim \sum_h a^{-D(h)}(a^h)^q = \sum_h a^{hq-D(h)} \tag{4}$$

The following equation is obtained by using equation (3) and equation (4) in combination, when $a$ approaches to $0^+$:

$$\tau(q) = \min_q(hq - D(h)) \tag{5}$$

Specifically, if $\tau(q)$ is continuously differentiable, the following equation can be obtained through Legendre transform:

$$\begin{cases} h(q) = \dfrac{d\tau(q)}{dq} \\ D(h(q)) = h(q)q - \tau(q) \end{cases} \tag{6}$$

The following equation is obtained by taking the logarithm of equation (4):

$$\log Z(q,a) = |b| + \tau(q)\log a$$

The gradient of this equation corresponds to the scale function with moment of order q; then, the distribution of $\tau(q)$ in relation to $q$ can be obtained by calculating the distribution of $Z(q, a)$ at different scales and performing least squares fitting. The following equation (7) is obtained by performing Taylor expansion on $\tau(q)$:

$$\tau(q) = -c_0 + c_1 q - \frac{c_2 q^2}{2} + \cdots \tag{7}$$

where, $c_n > 0$.

Specifically, a multifractal spectrum, i.e., a curve of $D(h)$ in relation to $h$, is obtained:

$$\begin{cases} h(q) = \dfrac{d\tau(q)}{dq} = c_1 - c_2 q \\ D\big(h(q)\big) = h(q)q - \tau(q) = c_0 - (h - c_1)^2/2c_2 \end{cases} \qquad (8)$$

Preferably, the number of the moment order q is set from -5 to 5, the step is set to 1, then altogether 11 spectrum coordinates are obtained, i.e., (h1, D1(h)), (h2, D2(h)), ..., (h9, D9(h)).

Preferably, the above 11 spectrum coordinates and cumulative coefficients $c_0$, $c_1$ and $c_2$ are used as fractal features. Features are extracted from multi-channel pulse wave signals one by one in the above-mentioned manner; here, 4 channels of pulse wave signals with different wavelength are used to calculate, and altogether 100 features are extracted.

In step 204, dimensionality reduction and clustering of feature space are performed. In this step, the pulse wave information of a certain number of diabetes patients and healthy persons are acquired as a database. The features are extracted with the method described in the step 203, principal component analysis is performed on the feature space to realize dimensionality reduction of features, several first principal components are selected according to the weights, on a condition that the sum of the weights of the features after dimensionality reduction accounts for 95% or more of the original feature space.

Preferably, the individual features are normalized before the principal component analysis, and the features after the principal component analysis and dimensionality reduction are clustered, and the data is classified into three classes by K-means or KNN clustering.

Preferably, the feature space after dimensionality reduction is clustered by unsupervised learning, with the number of clusters equal to 3. The distance of each data item to the center of each of the three clusters is logged; the distances of feature data item $j$ to the centers of the three clusters are $d_1^j$, $d_2^j$ and $d_3^j$.

In this embodiment, optical signals of the pulse wave are acquired on an empty stomach and at 2 hours after meal, and the disease information of the subject are obtained, including whether the subject suffers the disease, the type of disease, and the age of disease, etc.

In step 205, a screening model is established. In this step, the screening model is trained by using unsupervised learning and supervised learning in combination.

Preferably, binary classification prediction models are established for the three classes of data respectively according to the clustering result in the step 204. The predicted probability of disease of a data item $j$ in a center model $m$ (m=1, 2, 3) is $p_m^j$.

In this embodiment, during the training of the screening model, each model is trained with Cosine KNN and SVM algorithms, and each model outputs a probability of disease.

In step 206, the trained models are used for predicting new samples. For new samples, the steps 201 to 204 are performed sequentially, and the probabilities of disease of the new samples are predicted with the three prediction models trained in the step 205 respectively, a diabetes screening result and a disease risk prediction result are obtained by weighting the distances of the features of the new samples to the centers of the clusters.

Preferably, the prediction results from the three models are weighted according to the distances of the new samples to the centers of the clusters, specifically:

$$d_{sum}^j = d_1^j + d_2^j + d_3^j \qquad (9)$$

$$P_j = \sum_{m=1}^{3} \left( \frac{d_{sum}^j - d_m^j}{2d_{sum}^j} \right) * p_m^j \qquad (10)$$

Where, is the sum of the distances of a new sample $j$ to the centers of the three clusters, and $P_j$ is the weighted predicted probability of the new sample $j$. If $P_j$ is greater than 0.5, it is judged that the subject is a diabetic patient, and $P_j$ is the probability of disease risk.

Next, the photoplethysmography-based non-invasive diabetes screening and disease risk prediction method of the present invention will be further detailed in a specific embodiment.

In this embodiment, signals are acquired from the subject on an empty stomach or at 2 hours after meal. The subject is requested to sit still for 5 minutes and any strenuous exercise is prevented before formal data acquisition. Optical signals are acquired with the device as shown in FIG. 1, and pulse wave signals S1, S2, ..., Sn are transferred to the microprocessor.

In this embodiment, two visible light wavelengths are selected to be 530 nm and 660 nm, and near infrared light wavelengths are selected to be 805 nm and 940 nm.

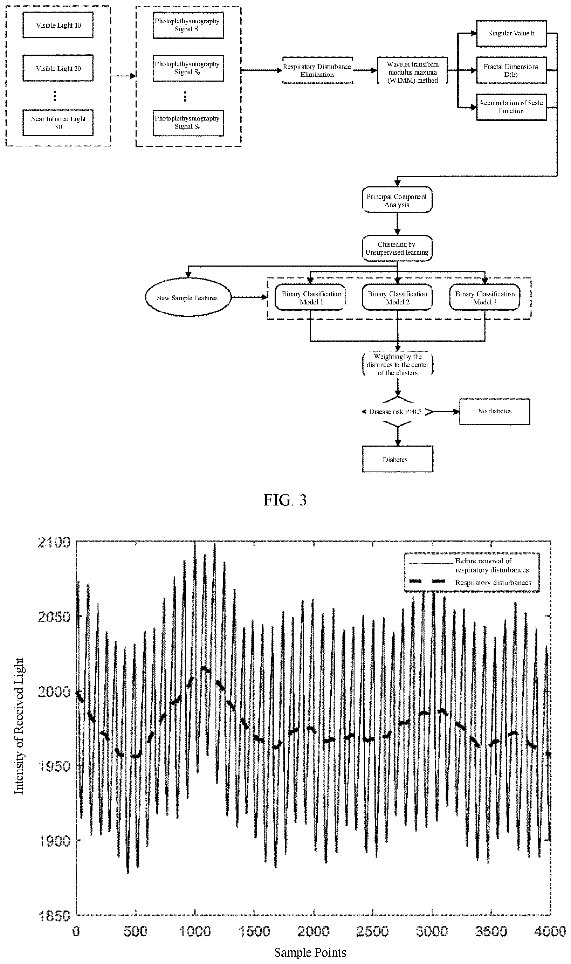

In this embodiment, the optical sensor includes a transmitter and a receiver, the transmitter is driven by an infrared driving circuit to act on the skin at 100 Hz frequency, the receiver is connected to a receiving detection circuit, and returns the detected pulse wave signal to the processor. The duration of action is not shorter than 45 seconds. Then, signal processing, multifractal spectrum calculation, modeling, and other operations are performed. FIG. 3 shows a flow chart of implementing the photoplethysmography-based non-invasive diabetes screening and disease risk prediction method according to this embodiment of the present invention. The specific steps of the method are as follows:

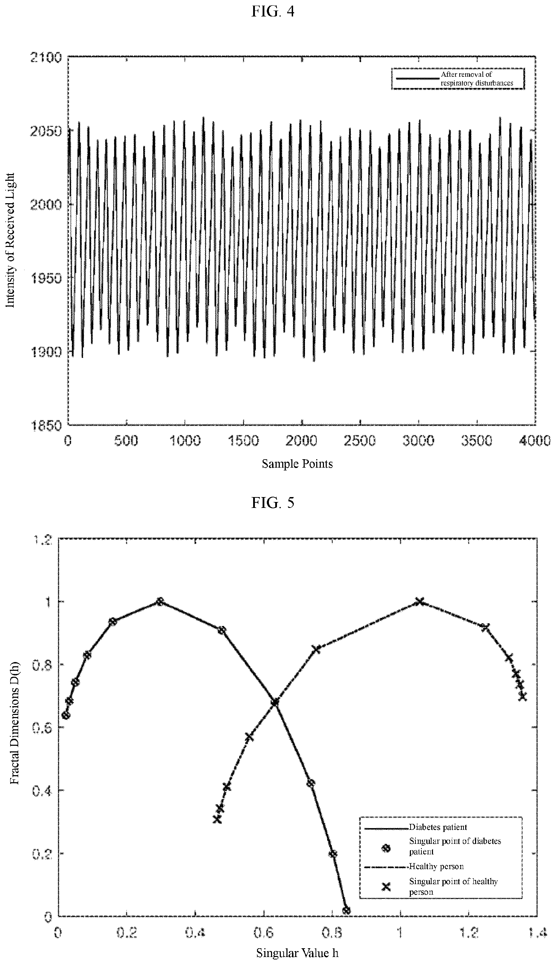

In step (1), low-frequency respiratory disturbances are eliminated. The obtained multi-band pulse wave signal is fed through a Butterworth low-pass filter, with a cut-off frequency set to 0.7Hz (or medium filtering is used, and the window width is 100 sample points) to obtain low-frequency respiratory disturbances, as shown in FIG. 4; the disturbances are eliminated from the raw signal to ensure that the DC component of the processed waveform is smooth and steady. The pulse wave signal after the respiratory disturbances are removed is shown in FIG. 5.

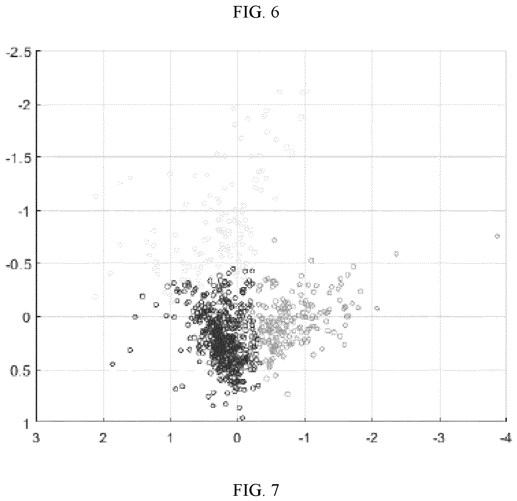

In step (2), multifractal spectrum features of the pulse wave signal are obtained on the basis of on wavelet transform. Multifractal analysis can describe the inherent law of a timing sequence waveform from a nonlinear aspect, and wavelet analysis is capable of characterizing a signal in time domain and frequency domain. Multifractal spectrum coordinates and cumulative coefficients are calculated with a wavelet transform modulus maxima (WTMM) method. FIG. 6 shows the multifractal spectrums of 660 nm red light pulse wave signals obtained from a healthy person and a diabetic patient respectively. The multifractal spectrums composed of 11 spectrum coordinates are quite different between the healthy person and the diabetic patient. The specific solving process is as follows:
The wavelet transform is set as follows:

$$W_{\psi}(t_0, a) = \frac{1}{a} \int_{-\infty}^{+\infty} f(t) \psi(\frac{t - t_0}{a}) dt \tag{1}$$

where $f(t)$ is the raw signal, $\psi(t)$ is a mother wavelet, and $a$ is a scale factor; it is proved that $W_{\psi}(t_0, a)$ is approximately equal to $a^{h(t0)}$ when $a$ approaches to $0^+$, where $h(t_0)$ is a singular index and represents the singularity of the wavelet transform.

The scale function and the multifractal spectrum are estimated with a wavelet transform modulus maxima (WTMM) method. Denote $N_h(a)$ is the number of wavelet transform modulus maxima lines with scale = $a$ and singular value = $h$, then $N_h(a)$ can be expressed by:

$$N_h(a) = a^{-D(h)} \tag{2}$$

$D(h)$ is the fractal dimensions having the same singular value $h$.

Preferably, a segmentation function of wavelet transform modulus maxima is defined as follows:

$$Z(q, a) = \sum_{l \in L(a)} |W_{\psi}(t_0, a)|^q \sim a^{\tau(q)} \tag{3}$$

$L(a)$ represents a set of modulus maxima lines with scale = $a$, $\tau(q)$ is the scale function, $q$ is the order of statistical moment; then, the following equation is obtained by substitute $N_h(a) = a^{-D(h)}$ into the above equation:

$$Z(q,a) \sim \sum_h a^{-D(h)}(a^h)^q = \sum_h a^{hq-D(h)} \tag{4}$$

The following equation is obtained by using equation (3) and equation (4) in combination, when *a* approaches to $0^+$:

$$\tau(q) = \min_q(hq - D(h)) \tag{5}$$

Furthermore, if $\tau(q)$ is continuously differentiable, the following equation can be obtained through Legendre transform:

$$\begin{cases} h(q) = \dfrac{d\tau(q)}{dq} \\ D(h(q)) = h(q)q - \tau(q) \end{cases} \tag{6}$$

The following equation is obtained by taking the logarithm of equation (4):

$$\log Z(q,a) = |b| + \tau(q)\log a$$

The gradient of this equation corresponds to the scale function at moment of order q; then, the distribution of $\tau(q)$ in relation to $q$ can be obtained by calculating the distribution of $Z(q, a)$ at different scales and performing least squares fitting. The following equation (7) is obtained by performing Taylor expansion on $\tau(q)$:

$$\tau(q) = -c_0 + c_1 q - \frac{c_2 q^2}{2} \tag{7}$$

where, $c_n > 0$.

Furthermore, a multifractal spectrum, i.e., a curve of $D(h)$ in relation to $h$, is obtained:

$$\begin{cases} h(q) = \dfrac{d\tau(q)}{dq} = c_1 - c_2 q \\ D(h(q)) = h(q)q - \tau(q) = c_0 - (h-c_1)^2/2c_2 \end{cases} \tag{8}$$

Preferably, the number of the moment order q is set from -5 to 5, the step is set to 1, then altogether 11 spectrum coordinates are obtained, i.e., (h1, D1(h)), (h2, D2(h)), ..., (h9, D9(h)).

Preferably, the above 11 spectrum coordinates and cumulative coefficients $c_0$, $c_1$ and $c_2$ are used as fractal features. Features are extracted from multi-channel pulse wave signals one by one in the above-mentioned manner; here, 4 channels of pulse wave signals with different wavelengths are used, and altogether 100 features are extracted.

In step (3), dimensionality reduction and clustering of feature space are performed. The pulse wave information of a certain number of diabetes patients and healthy persons are acquired as a database. The features are extracted with the method described in the step (2), principal component analysis is performed on the feature space to realize feature dimensionality reduction; the individual features are normalized before the principal component analysis with a requirement that the sum of the weights of the features after dimensionality reduction accounts for 95% or more of the original feature space. The feature space after dimensionality reduction is clustered by K-means or KNN unsupervised learning, with the number of clusters equal to 3. The distance of each data item to the center of each of the three clusters is logged; specifically, the distances of a data item *j* to the centers of the three clusters are, and respectively, as shown in FIG. 7; in the view of first and second principal components, the data is classified into three classes through clustering.

In step (4), a screening model is established. The screening model is trained by using unsupervised learning and supervised learning in combination. Binary classification prediction models are established for the three classes of data respectively according to the clustering result in the step (4). The predicted probability of disease of a data item *j* in a center model *m* (m=1, 2, 3) is $p^j_m$.

In step (5), the trained models are used for predicting new samples. For new samples, the steps (1) to (3) are executed sequentially, and the probabilities of disease of the new samples are predicted respectively with the three prediction models trained in the step (4), and the distances of the features of the new samples to the centers of the clusters are weighted:

$$d^j_{sum} = d^j_1 + d^j_2 + d^j_3 \tag{9}$$

$$P_j = \sum_{m=1}^{3} \left( \frac{d^j_{sum} - d^j_m}{2d^j_{sum}} \right) * p^j_m \tag{10}$$

Where, is the sum of the distances of a new sample *j* to the centers of the three clusters, and $P_j$ is the weighted predicted probability of the new sample *j*.

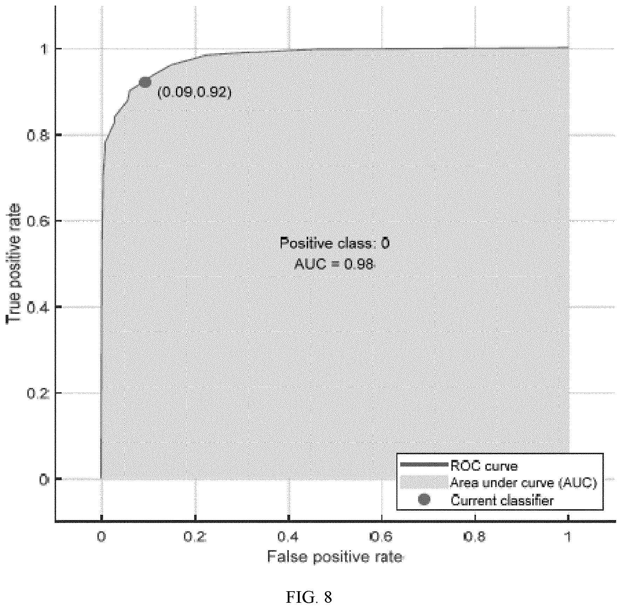

If $P_j$ is greater than 0.5, it is judged that the subject is a diabetic patient, and $P_j$ is the probability of disease risk. The accuracy of diabetes prediction reaches about 92%, as shown in FIG. 8.

In this embodiment, through the above process, after the database is generated and the screening and disease risk prediction models are completed, the user can obtain a prediction result quickly within 1 minute. The method in this embodiment can be integrated as a health management means into a wearable carrier, such as a wrist strap or fingertip probe, etc.

The photoplethysmography-based non-invasive diabetes screening and prediction system and method according to the present invention provide a red light-near infrared light sensor system to be placed on the skin of a human body, which can be used at different body sites such as fingertips and wrists, etc., and includes two or more visible light sensors and two or more near infrared light sensors. The red light sensors and near infrared light sensors are utilized to obtain a photoplethysmography signal from the human body, multifractal spectrum features are obtained through wave processing and feature extraction, and a diabetes screening model is established, so as to realize non-invasive, portable and wearable diabetes screening and disease risk prediction.

Those skilled in the art should understand: the embodiments described above are only some preferred embodiments of the present invention, and should not be deemed as constituting any limitation to the present invention. Although the present invention is described and illustrated in detail with reference to the embodiments mentioned above, those skilled in the art can easily make modifications to the technical solution described above in the embodiments or make equivalent replacement of some technical features. Any modification, equivalent replacement, or improvements made to the embodiments without departing from the spirit and the principle of the present invention shall be deemed as falling into the scope of protection of the present invention.

**Claims**

1. A photoplethysmography-based non-invasive diabetes prediction system, comprising an optical signal emitter, a driving circuit, a receiving circuit, and a processor module, wherein,

    the optical signal emitter is driven by the driving circuit to obtain and emit an optical signal having a fixing wavelength and intensity;
    the receiving circuit converts the optical signal of the received photoplethysmography into an electrical signal of the photoplethysmography, performs amplification, filtering, digital-analog conversion, and sends a digital photoplethysmography signal to the processor module; and
    the processor module performs signal processing, feature extraction, modeling and prediction on the digital photoplethysmography signal, and visually displays the same.

2. The photoplethysmography-based non-invasive diabetes prediction system according to claim 1, wherein the optical signal emitter further comprises a visible light transmitter having two or more wavelengths and a near-infrared light transmitter having two or more wavelengths.

3. The photoplethysmography-based non-invasive diabetes prediction system according to claim 1, wherein the receiving circuit comprises an optical signal receiver, an amplifier filter circuit, and an analog-digital converter circuit, wherein,

the optical signal receiver converts the optical signal of the received photoplethysmography into the electrical signal of the photoplethysmography and sends the electrical signal of the photoplethysmography to the amplifier filter circuit;
the amplifier filter circuit performs amplification and filtering on the electrical signal of the photoplethysmography to eliminate high-frequency noises; and
the analog-digital converter circuit converts the electrical signal of the photoplethysmography into a digital photoplethysmography signal and then sends the digital photoplethysmography signal to the processor module.

4. The photoplethysmography-based non-invasive diabetes prediction system according to claim 1, wherein, the processor module eliminates low-frequency respiratory disturbances, obtains multifractal spectrum features from the digital photoplethysmography signal based on wavelet transform, performs dimensionality reduction and clustering of feature space, establishes and trains a screening model, and performs prediction of new samples according to the screening model.

5. The photoplethysmography-based non-invasive diabetes prediction system according to claim 4, wherein, the processor module calculates multifractal spectrum coordinates and cumulative coefficients by using a wavelet transform modulus maxima (WTMM) method, and obtains multifractal spectrum features from the digital photoplethysmography signal.

6. The photoplethysmography-based non-invasive diabetes prediction system according to claim 4, wherein the processor module extracts features from a generated photoplethysmography information database and performs normalization on the features, and performs principal component analysis on the feature space to realize feature dimensionality reduction; clusters the feature space after dimensionality reduction by K-means or KNN unsupervised learning , sets a number of clusters, logs the distance of each data item to the center of each cluster, and classifies the data according to the number of clusters.

7. The photoplethysmography-based non-invasive diabetes prediction system according to claim 4, wherein the processor module trains the screening model by using unsupervised learning and supervised learning in combination.

8. A photoplethysmography-based non-invasive diabetes prediction method, comprising the following steps:

1) obtaining a pulse wave signal of human body;
2) eliminating low-frequency respiratory disturbances from the pulse wave signal;
3) obtaining multifractal spectrum features of the pulse wave signal on the basis of wavelet transform;
4) performing dimensionality reduction and clustering of feature space of the pulse wave signal;
5) establishing a screening model; and
6) performing prediction of new samples according to the screening model.

9. The photoplethysmography-based non-invasive diabetes prediction method according to claim 8, wherein the step 3) further comprises calculating multifractal spectrum coordinates and cumulative coefficients by using a wavelet transform modulus maxima (WTMM) method.

10. The photoplethysmography-based non-invasive diabetes prediction method according to claim 8, wherein the step 3) further comprises the following steps:

defining a wavelet transform function, and calculating a singular index of wavelet transform according to the pulse wave signal, a mother wavelet, and a scale factor;
setting a scale according to the scale factor, and calculating fractal dimensions having a same singular value according to the scale and a number of wavelet transform modulus maxima lines;

defining a segmentation function of wavelet transform modulus maxima, substituting a set of scale modulus maxima line, an order and a scale of statistical moments into the segmentation function, and calculating a scale function;

performing Legendre transform on the scale function, calculating a distribution of the segmentation function for wavelet transform modulus maxima at different scales, and performing least squares fitting to obtain the distribution of the moment order corresponding to the scale function; and

performing Taylor expansion on the scale function to obtain a multifractal spectrum.

11. The photoplethysmography-based non-invasive diabetes prediction method according to claim 10, wherein the step of performing Taylor expansion on the scale function to obtain a multifractal spectrum further comprises obtaining spectrum coordinates of fixed-step moment order, and using the spectrum coordinates and the cumulative coefficients as fractal features.

12. The photoplethysmography-based non-invasive diabetes prediction method according to claim 8, wherein the step 4) further comprises generating a photoplethysmography information database, extracting features and performing normalization on the features, and performing principal component analysis on the feature space to realize feature dimensionality reduction.

13. The photoplethysmography-based non-invasive diabetes prediction method according to claim 12, wherein clustering the feature space after dimensionality reduction by K-means or KNN unsupervised learning, setting a number of clusters, logging the distance of each data item to the center of each cluster, and classifying the data according to the number of clusters.

14. The photoplethysmography-based non-invasive diabetes prediction method according to claim 8, wherein the step 5) further comprises establishing binary classification prediction models for the clustering results respectively.

15. The photoplethysmography-based non-invasive diabetes prediction method according to claim 4, wherein the step 6) further comprises weighting distances of the features of new samples to the centers of the clusters, and predicting a probability according to the weighting result.

Green Light LED 111

Red Light LED 112

Visible Light Signal Emitter
11

Near-Infrared Light
LED 121

Near-Infrared Light
LED 122

Near-Infrared Light Signal
Emitter 12

Optical Signal Emitter 10

Driving Circuit

30

Receiving Circuit 40

Optical Signal
Receiver 401

Amplifier
Filter Circuit
402

Analog-Digital
Converter
Circuit 403

Processor
Module
50

FIG. 1

Obtain a pulse wave signal of human
body                                          201

Eliminate low-frequency respiratory
disturbances                                  202

Obtain multifractal spectrum features of the pulse wave signal
on the basis of wavelet transform             203

Perform dimensionality reduction and clustering of feature
space                                         204

Establish a screening model                   205

Use the trained screening model for prediction of new samples   206

FIG. 2

FIG. 3

FIG. 4

FIG. 5

FIG. 6

FIG. 7

FIG. 8

EP 4 218 569 A1

# INTERNATIONAL SEARCH REPORT

International application No.

**PCT/CN2021/118986**

### A. CLASSIFICATION OF SUBJECT MATTER

A61B 5/1455(2006.01)i

According to International Patent Classification (IPC) or to both national classification and IPC

### B. FIELDS SEARCHED

Minimum documentation searched (classification system followed by classification symbols)

A61B5/-

Documentation searched other than minimum documentation to the extent that such documents are included in the fields searched

Electronic data base consulted during the international search (name of data base and, where practicable, search terms used)

CNABS, WPI, EPODOC, CNKI: 光电, 容积, 脉搏, 血糖, 糖尿病, 建模, 小波变换, 预测, photoelectric, volume, pulse, wave, blood, sugar, glucose, model, predict+

### C. DOCUMENTS CONSIDERED TO BE RELEVANT

| Category* | Citation of document, with indication, where appropriate, of the relevant passages | Relevant to claim No. |
|---|---|---|
| PX | CN 112120711 A (GLOBAL HEALTH ARK MEDICAL TECHNOLOGY (BEIJING) CO., LTD.) 25 December 2020 (2020-12-25)<br>claims 1-15 | 1-15 |
| X | CN 107374646 A (INSTITUTE OF SEMICONDUCTORS, CHINESE ACADEMY OF SCIENCES) 24 November 2017 (2017-11-24)<br>description, paragraphs [0041]-[0087], and figures 1-5 | 1-15 |
| X | CN 110623678 A (SHENZHEN YOUGE TECHNOLOGY CO., LTD. et al.) 31 December 2019 (2019-12-31)<br>description, paragraphs [0023]-[0090], and figures 1-5 | 1-15 |
| X | CN 107296616 A (QIANHAI ANYCHECK INFORMATION TECHNOLOGY COMPANY) 27 October 2017 (2017-10-27)<br>description, paragraphs [0005]-[0059], and figures 1-5 | 1-15 |
| X | CN 105193423 A (PEKING UNIVERSITY SHENZHEN GRADUATE SCHOOL) 30 December 2015 (2015-12-30)<br>description, paragraphs [0022]-[0052], and figures 1-8 | 1-15 |
| A | WO 2020099218 A1 (MY-VITALITY S.A.R.L.) 22 May 2020 (2020-05-22)<br>entire document | 1-15 |

☐ Further documents are listed in the continuation of Box C. ☑ See patent family annex.

| | | |
|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" | document member of the same patent family |

| Date of the actual completion of the international search | Date of mailing of the international search report |
|---|---|
| **30 November 2021** | **15 December 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.

**PCT/CN2021/118986**

| Patent document cited in search report | | | Publication date (day/month/year) | Patent family member(s) | | | Publication date (day/month/year) |
|---|---|---|---|---|---|---|---|
| CN | 112120711 | A | 25 December 2020 | None | | | |
| CN | 107374646 | A | 24 November 2017 | CN | 107374646 | B | 30 October 2020 |
| CN | 110623678 | A | 31 December 2019 | None | | | |
| CN | 107296616 | A | 27 October 2017 | WO | 2018214297 | A1 | 29 November 2018 |
| CN | 105193423 | A | 30 December 2015 | None | | | |
| WO | 2020099218 | A1 | 22 May 2020 | CA | 3119887 | A1 | 22 May 2020 |

Form PCT/ISA/210 (patent family annex) (January 2015)

## REFERENCES CITED IN THE DESCRIPTION

**Patent documents cited in the description**

- CN 107680676 A

- CN 106682412 A